# EUROPEAN PATENT SPECIFICATION

(11) **EP 4 042 963 B1**
(45) Date of publication and mention of the grant of the patent: **28.02.2024**
(21) Application number: 20876074.4
(22) Date of filing: 18.09.2020
(51) Int. Cl.: A61B 34/00, A61B 34/30

(54) **SURGICAL TOOL**
CHIRURGISCHES INSTRUMENT
INSTRUMENT CHIRURGICAL

(30) Priority: 17.10.2019 JP 2019190340
(43) Date of publication of application: 17.08.2022
(73) Proprietor: RIVERFIELD Inc., 1070052 Tokyo (JP)
(72) Inventor: SHINDO, Koki, Tokyo 160-0017 (JP); TADANO, Kotaro, Tokyo 160-0017 (JP)
(74) Representative: adares Patent- und Rechtsanwälte Reininger & Partner GmbB
(86) International application number: PCT/JP2020/035625
(87) International publication number: WO 2021/075212

(56) References cited:
- EP-A2- 2 620 118
- WO-A1-2016/119972
- WO-A1-2018/193500
- JP-A- 2006 191 939
- JP-A- 2018 191 881
- US-A1- 2011 277 580
- US-A1- 2018 168 681

## Description

### TECHNICAL FIELD

The present disclosure relates to a surgical tool used for medical robots.

### BACKGROUND ART

In recent years, medical treatments using robots have been proposed for the purpose of reducing burdens on operators and reducing manpower at medical facilities. In the field of surgery, proposals have been made with regard to medical robots in which operators treat patients with a multi-degree-of-freedom manipulator having a remotely operable arm with multi-degree of freedom (see, for example, Patent Document 1).

Patent Document 1 discloses a configuration that allows attachment/detachment of a surgical tool used for treatment to/from a medical robot. Patent Document 1 also discloses a configuration in which a driving force in a linear motion direction is transmitted from the medical robot to the surgical tool, and the driving force is transmitted through a transmission member, such as a wire and a rod, arranged within the surgical tool to a treatment portion, such as a gripper, arranged at an end of the surgical tool.

US 2018/168681 A1 discloses a tool assembly for a robot comprising a yoke coupled pivotally to a lever arm having one end coupled to a pivot joint and another end coupled to an actuator or linear input coupling. The yoke can include an elongated body that extends generally parallel to a housing of the tool assembly, wherein the yoke can be positioned within and slidably disposed relative to the housing and coupled to an inner closure tube such that when the yoke is caused to translate, the inner closure tube is caused to translate thereby pivoting a clamp arm between open and closed configurations relative to a blade. US 2011/277580 A1 describes a surgical instrument for use with a robotic manipulator having a force transmission mechanism. The force transmission mechanism has a lever arm which is supported by a pivot between a first end and a second end, a coupler link, and output link.

WO 2016/119972 A1 describes a surgical tool for manual operation. The surgical tool contains a shaft part, which is fixedly connected at one end to a shaft grip, a sliding part, at least two lever arms each articulated on at least two shaft articulation points spaced apart from one another on the shaft part or on the shaft grip, and a coupling section which connects the at least two lever arms and coupling joints to one another such that same can pivot in the same direction, the coupling joints being spaced apart from one another.

### PRIOR ART DOCUMENT

### PATENT DOCUMENT

Patent Document 1: Japanese Unexamined Patent Application Publication No. 2018-191881

### SUMMARY OF THE INVENTION

### PROBLEMS TO BE SOLVED BY THE INVENTION

There has been a trend to require recent treatment for patients to have a low invasiveness, in which the degree of invasiveness to patients is reduced, and to provide an improved cosmetic outcome. In order to meet these demands, further miniaturization and narrowing in diameter of surgical tools of master-slave type medical robots have been desired.

At the same time, operators of such medical robots have been required to spend less time to learn and acquire an operation procedure of a medical robot and to be able to stably and smoothly handle the surgical tool as intended.

For example, it has been desired to improve the accuracy in estimating the magnitude, the direction, and so on of an external force acting on the surgical tool based on information, such as the position and the driving force of an actuator that drives the surgical tool, and transmit the estimated external force to an operator remotely handling the surgical tool. The accuracy in estimating an external force depends on the S/N ratio when the external force is detected and the resolution when an operation amount of the treatment portion is measured. The S/N ratio mentioned herein is also referred to as a signal-to-noise ratio.

When the size of the treatment portion, such as a gripper, of the surgical tool is changed, the amount of operation of the treatment portion varies even though strokes generated by the driving force transmitted from the medical robot to the surgical tool have the same lengths. In addition, the magnitude of a force generated in the treatment portion also varies even though the magnitude of the driving force is the same.

Hence, when the size of the surgical tool attached to the medical robot, that is, for example, the size of the treatment portion such as a gripper, is changed, the aforementioned S/N ratio and resolution also vary, affecting the accuracy in estimating an external force. In other words, problems have been found in that it becomes difficult to stably and smoothly operate the surgical tool as intended, and difficult to inhibit deterioration in accuracy in estimating the external force.

It is desirable that one aspect of the present disclosure provides a surgical tool with which it is possible to inhibit deterioration in operability and accuracy in estimating an external force due to a change in size of the surgical tool.

### MEANS FOR SOLVING THE PROBLEMS

The problem is solved by a surgical tool according to claim 1.

The surgical tool according to one aspect of the present disclosure comprises a driven portion configured to move upon receipt of an external driving force for moving in a linear motion direction, a power transmission portion configured to transmit the driving force for moving in the linear motion direction to a treatment portion configured to perform a medical treatment, a conversion portion configured to convert an amount of movement of the driven portion in the linear motion direction to transmit the amount of movement converted to the power transmission portion, and a body storing therein the driven portion and the conversion portion and supporting the treatment portion.

With such a configuration, the provision of the conversion portion enables the amount of movement of the driven portion in the linear motion direction to be converted and transmitted to the power transmission portion. For example, it is possible to set the ratio of conversion by the conversion portion depending on the size of, for example, the treatment portion. Specifically, in the case where the size of the treatment portion is relatively small, a conversion ratio is set to reduce the amount of movement produced by the driving force, and in the case where the size of the treatment portion is relatively large, a conversion ratio is set to increase the amount of movement produced by the driving force.

This makes it easier, even when the size of, for example, the treatment portion is changed, to maintain the relationship between the amount of movement transmitted from outside to the driven portion and the amount of operation of the treatment portion. This also makes it possible to inhibit fluctuations in the S/N ratio and fluctuations in the resolution due to a change in size of, for example, the treatment portion, thus enabling smooth control of operation of the treatment portion and inhibiting deterioration in accuracy in estimating the external force applied to the treatment portion.

As a result, it is easier to achieve safety and to inhibit complications in robot surgery with the surgical tool according to one aspect of the present disclosure. In addition, this facilitates improvement in QOL of patients and reduction of burden on doctors during surgery. The term QOL used herein is an abbreviation for quality of life. Furthermore, this facilitates improvement in the learning curve in robot surgery with the surgical tool according to one aspect of the present disclosure.

In another aspect of the present disclosure, the surgical tool further comprises a connection portion arranged between the power transmission portion and the conversion portion, and the connection portion is configured to transmit the driving force transmitted from the conversion portion to the power transmission portion.

The provision of the connection portion makes transmission of the driving force from the conversion portion to the power transmission portion easier as compared to a case where the driving force is directly transmitted to the power transmission portion. This also makes the setting easier for converting the amount of movement in the linear motion direction into a specified converted amount.

In the above-described configuration, the conversion portion is formed in an elongated shape comprising a first end rotatably supported with respect to the driven portion and a second end rotatably supported with respect to a supporting portion supporting the conversion portion, and the connection portion is rotatably supported with respect to the conversion portion between the first end and the second end.

Forming the conversion portion into the shape described above makes the setting easier for reducing the amount of movement in the linear motion direction at a specified conversion ratio. In other words, it is possible to change the conversion ratio of the amount of movement in the linear motion direction by changing the ratio between a distance from the position at which the conversion portion is rotatably supported with respect to the driven portion to the position at which the connection portion is rotatably supported, and a distance from the position at which the conversion portion is rotatably supported with respect to the support portion to the position at which the connection portion is rotatably supported.

In the above-described configuration, according to a non-inventive embodiment the conversion portion is formed in an elongated shape comprising a first end rotatably supported with respect to the driven portion and a second end rotatably supported with respect to the connection portion, and a portion of the conversion portion between the first end and the second end is rotatably supported with respect to a support portion supporting the conversion portion.

Forming the conversion portion into the shape described above makes the setting easier for reducing the amount of movement in the linear motion direction or for increasing the amount of movement at a specified conversion ratio. In other words, it is possible to change the conversion ratio of the amount of movement in the linear motion direction by changing the ratio between a distance from the position at which the conversion portion is rotatably supported with respect to the driven portion to the position at which the conversion portion is rotatably supported with respect to the support portion, and a distance from the position at which the conversion portion is rotatably supported with respect to the connection portion to the position at which the conversion portion is rotatably supported with respect to the support portion.

In the above-described configuration, according to a non-inventive embodiment in the conversion portion, a first distance from a position at which the conversion portion is rotatably supported with respect to the driven portion to a position at which the support portion is rotatably supported is greater than a second distance from a position at which the conversion portion is rotatably supported with respect to the connection portion to the position at which the support portion is rotatably supported.

Setting the first distance greater than the second distance in the conversion portion in this manner enables the amount of movement of the driven portion in the linear motion direction to be converted into a smaller amount and transmitted to the power transmission portion. Moreover, the magnitude of the driving force is converted into a larger magnitude to be transmitted to the power transmission portion.

In the above-described configuration, according to a non-inventive embodiment in the conversion portion, a first distance from a
position at which the conversion portion is rotatably supported with respect to the driven portion to a position at which the support portion is rotatably supported is smaller than a second distance from a position at which the conversion portion is rotatably supported with respect to the connection portion to the position at which the support portion is rotatably supported.

Setting the first distance smaller than the second distance in the conversion portion in this manner enables the amount of movement of the driven portion in the linear motion direction to be converted into a larger amount and transmitted to the power transmission portion. Moreover, the magnitude of a driving force is converted into a smaller magnitude to be transmitted to the power transmission portion.

### EFFECTS OF THE INVENTION

According to the surgical tool of the present disclosure, the provision of the conversion portion enables the amount of movement of the driven portion in the linear motion direction to be converted and transmitted to the power transmission portion, thus achieving an effect that makes it possible to inhibit deterioration in operability and accuracy in estimating an external force due to a change in size of the surgical tool.

### BRIEF DESCRIPTION OF THE DRAWINGS

FIG. 1 is a diagram illustrating an overall configuration of a surgical tool according to a first embodiment of the present disclosure.
FIG. 2 is a diagram illustrating a configuration of an attachment/detachment surface of a body of the surgical tool in FIG. 1.
FIG. 3 is a diagram illustrating configurations of a driven portion, a connection portion, a conversion portion, and a power transmission portion.
FIG. 4 is a perspective view illustrating the configurations of the driven portion, the connection portion, the conversion portion, and the power transmission portion in FIG. 3.
FIG. 5 is a diagram illustrating the configuration of the connection portion in FIG. 3.
FIG. 6 is a diagram illustrating transmission of a driving force performed by the driven portion, the connection portion, the conversion portion, and the power transmission portion in FIG. 3.
FIG. 7 is another diagram illustrating transmission of a driving force performed by the driven portion, the connection portion, the conversion portion, and the power transmission portion in FIG. 3.
FIG. 8 is a diagram illustrating configurations of a driven portion, a connection portion, a conversion portion, and a power transmission portion of a surgical tool according to a second embodiment of the present disclosure.
FIG. 9 is a diagram illustrating the configuration of the connection portion in FIG. 8.

### EXPLANATION OF REFERENCE NUMERALS

1, 101... surgical tool, 10...body, 21, 121...driven portion, 31, 131...conversion portion, 51...power transmission portion, 70...forceps (treatment portion), D21...first distance, D22...second distance

### MODE FOR CARRYING OUT THE INVENTION

### [First Embodiment]

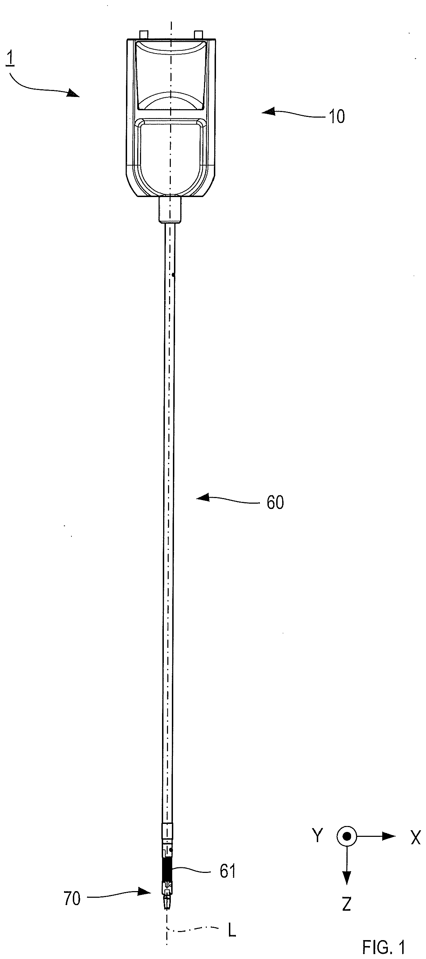

A description will be given of a surgical tool 1 in a first embodiment of the present disclosure with reference to FIG. 1 to FIG. 7. The surgical tool 1 of the present embodiment is used for master-slave type surgical robots. In the present embodiment, an end effector which is arranged at the leading end of a shaft 60 extending from a body 10 is a pair of forceps 70.

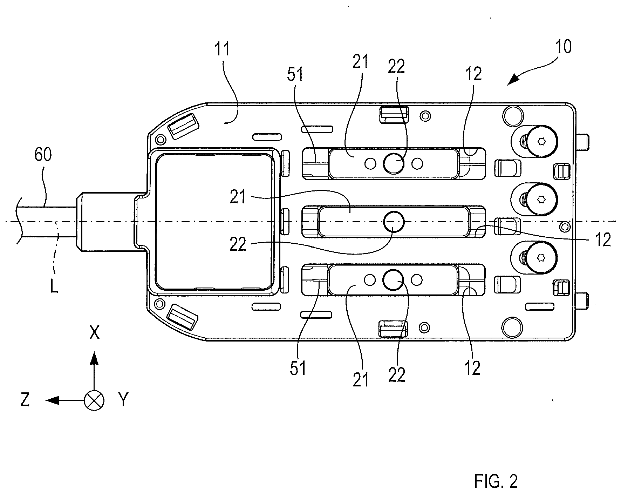

As shown in FIG. 1 and FIG. 2, the surgical tool 1 comprises the body 10 having a storage space therein, the shaft 60 extending in a rod shape from the body 10, and the forceps 70 arranged at the end of the shaft 60 opposite from the body 10. The pair of forceps 70 corresponds to one example configuration of the treatment portion.

In the present embodiment, for the purpose of simplifying the description, a direction along an axis line L of the shaft 60 will be described as a Z-axis, and a direction from the body 10 toward the forceps 70 will be described as a positive direction of the Z-axis. A direction orthogonal to the Z-axis and parallel to a paper surface of FIG. 1 will be described as an X-axis, and a rightward direction with respect to the positive direction of the Z-axis will be described as the positive direction of the X-axis. A direction orthogonal to the X-axis and the Z-axis will be described as a Y-axis, and a direction from a paper surface of FIG. 1 toward a viewer will be described as a positive direction of the Y-axis.

The body 10 is a portion of the surgical tool 1 that is attached/detached to/from a master-slave type surgical robot, and also a portion supporting the shaft 60. As shown in FIG. 2, an attachment/detachment surface 11 of the body 10, which is attached/detached to/from the surgical robot, comprises driven side slits 12 that are elongated holes extending along a Z-axis direction. In the present embodiment, the surface located on the side in the negative direction of the Y-axis is the attachment/detachment surface 11.

In the driven side slits 12, driven portions 21, which will be described later, are arranged to be linearly movable with respect to the body 10 along the Z-axis direction. In the present embodiment, three driven side slits 12 are aligned at intervals along an X-axis direction. The lengths of the three driven side slits 12 along the Z-axis direction may be the same, or the lengths of two of the driven side slits 12 may be the same and the length of one of the driven side slits 12 may be different, or the lengths of all the driven side slits 12 may be different. The number of the driven side slits 12 that the body 10 comprises may be more than three, or may be less than three.

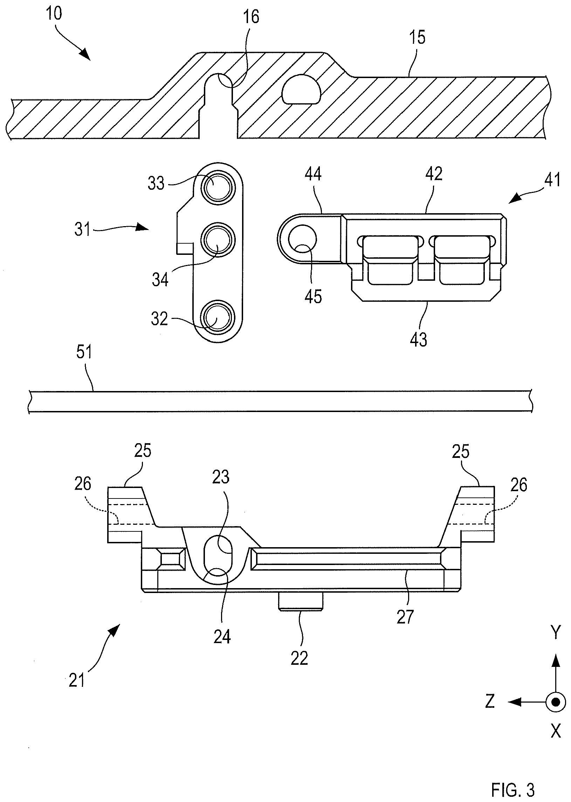

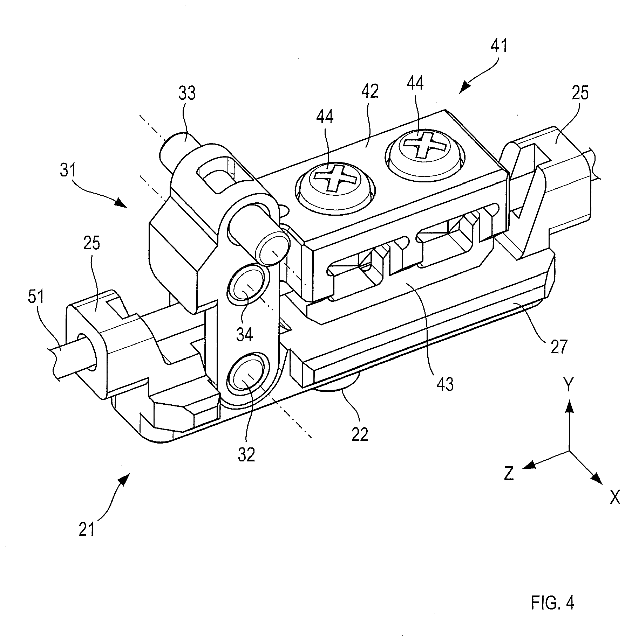

As shown in FIG. 3 and FIG. 4, the body 10 comprises, inside thereof, driven portions 21 used for transmitting a driving force for moving, for example, the forceps 70, conversion portions 31, connection portions 41, and power transmission portions 51.

The driven portions 21 receive from the surgical robot a driving force for moving the forceps 70 and the like. As shown in FIG. 2, the driven portions 21 are arranged to be linearly movable within the driven side slits 12 along the Z-axis direction in accordance with the driving force transmitted from the surgical robot (see.).

Each of the driven portion 21 comprises a protrusion 22, driven side openings 23, guide portions 25, and slide guides 27.

The protrusion 22 is a columnar portion that protrudes from the driven portion 21 in the negative direction of the Y-axis, and, when the driven portion 21 is arranged within the driven side groove 12, the protrusion 22 protrudes in the negative direction of the Y-axis further than the attachment/detachment surface 11. The protrusion 22 is configured to engage with a recess formed in a component of the surgical robot that transmits a driving force. This engagement allows transmission of the driving force for producing linear motion along the Z-axis direction.

As shown in FIG. 3, the driven side openings 23 are connected to the conversion portion 31 such that the conversion portion 31 is rotatable. Each of the driven side opening 23 is a through-hole which extends along the X-axis direction and accommodates a driven shaft 32 of the conversion portion 31 inserted therein. A cross-section of the driven side opening 23, that is a cross-section cut along a plane orthogonal to the X-axis, has an oval shape with its long axis extending along the Y-axis direction. A cutout portion 24 may be formed around the driven side opening 23 of the driven portion 21 to allow rotational movement of the conversion portion 31.

As shown in FIG. 3 and FIG. 4, each of the guide portions 25 has a through hole 26 in which the power transmission portion 51 is inserted. A gap is formed between the through hole 26 and the power transmission portion 51, allowing relative movement between the driven portion 21 and the power transmission portion 51 along the Z-axis direction. In the present embodiment, the guide portions 25 are formed at the end of the driven portion 21 in the positive direction and the end in the negative direction of the Z-axis, protruding in the positive direction of the Y-axis.

The slide guides 27 protrude from the driven portion 21 in the positive direction and the negative direction of the X-axis, and each has a ridge shape extending along the Z-axis direction. The slide guides 27 are engaged with grooves or step shapes formed inside the driven side slit 12 along the Z-axis direction so as to guide movement of the driven portion 21 along the driven side slit 12.

The conversion portion 31 forms a linkage mechanism or a lever mechanism that transmits a driving force transmitted to the driven portion 21 to the connection portion 41. The linkage mechanism or the lever mechanism comprising the conversion portion 31 of the present embodiment reduces an amount of movement of the driven portion 21 along a linear motion direction, which is along the Z-axis direction, and transmits the reduced amount of movement to the connection portion 41. The linkage mechanism or the lever mechanism also increases the magnitude of a driving force in the driven portion 21 and transmits the driving force with the increased magnitude to the connection portion 41.

The conversion portion 31 is an elongated member extending at least along the Y-axis direction. In one end portion of the conversion portion 31 on a negative direction side of the Y-axis, that is in the end portion adjacent to the driven portion 21, a columnar driven shaft 32 is arranged to extend along the X-axis direction. In the end portion of the conversion portion 31 on a positive direction side of the Y-axis, a columnar support shaft 33 supporting the conversion portion 31 is arranged to extend along the X-axis direction. Between the driven shaft 32 and the support shaft 33 of the conversion portion 31, a columnar connection shaft 34 is arranged to extend along the X-axis direction.

The end portion of the conversion portion 31 adjacent to the driven portion 21 has a bifurcated shape in which the bifurcated ends thereof are spaced apart along the X-axis direction and extend in the negative direction of the Y-axis. Between the bifurcated ends, a portion of the driven portion 21 in which the driven side opening 23 and the cutout portion 24 are formed is inserted. In an area of the conversion portion 31 in which the connection shaft 34 is arranged, a recess that is open in the negative direction of the Z-axis is formed to receive part of the connection portion 41.

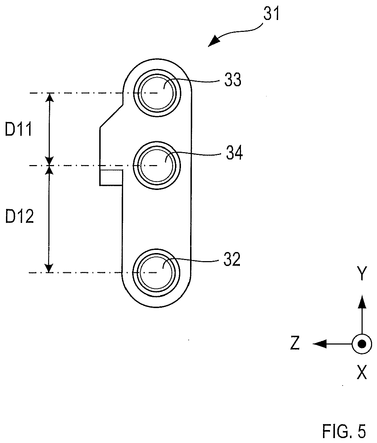

As shown in FIG. 5, in the conversion portion 31, the distance from the center of the support shaft 33 to the center of the connection shaft 34 is a first distance D 11. Moreover, in the conversion portion 31, the distance from the center of the driven shaft 32 to the center of the connection shaft 34 is a second distance D12.

As shown in FIG. 4, the support shaft 33 is arranged to extend in the positive direction and the negative direction of the X-axis beyond the conversion portion 31. As shown in FIG. 3, the support shaft 33 is arranged in a support groove 16 of a support portion 15 of the body 10. The support groove 16 is open in the negative direction of the Y-axis and extends along the X-axis direction.

As shown in FIG. 3 and FIG. 4, the connection portion 41 transmits a driving force transmitted from the conversion portion 31 to the power transmission portion 51. The connection portion 41 comprises a first connection portion 42, a second connection portion 43, and securing portions 44.

The first connection portion 42 and the second connection portion 43 hold and fasten the power transmission portion 51 therebetween. The securing portions 44 join the first connection portion 42 and the second connection portion 43, and are screws in the present embodiment. The securing portions 44 may be screws as mentioned above, or may have other structures used for fastening.

The first connection portion 42 comprises a protruding portion 45 inserted to the recess formed around the portion of the conversion portion 31 in which the connection shaft 34 is arranged. The protruding portion 45 comprises a connection hole 46 that is a through hole extending along the X-axis direction. The connection shaft 34 of the conversion portion 31 is rotatably inserted in the connection hole 46.

The power transmission portion 51 transmits a driving force transmitted from the connection portion 41 to the forceps 70. The power transmission portion 51 is arranged to extend from the inside of the body 10 to the forceps 70 through the shaft 60.

In the present embodiment, the power transmission portion 51 is a wire which is a cord-shaped element. The power transmission portion 51 may be entirely formed of a wire, or may be, for example, but not limited to, a combination of a columnar or cylindrical rod in one part and a wire.

As shown in FIG. 1, the shaft 60 is a cylindrically formed member arranged to extend from the body 10 along the Z-axis direction. The forceps 70 are arranged in the end portion of the shaft 60 in the positive direction of the Z-axis. The shaft 60 comprises a joint portion 61 near the forceps 70.

The joint portion 61 is configured to allow changes in orientation of the forceps 70, and is rotatable about a rotational axis in the X-axis direction and a rotational axis in the Y-axis direction. The joint portion 61 is configured to be rotated by, for example, a driving force transmitted by the power transmission portion 51. The configuration of the joint portion 61 is not limited to a particular configuration.

The forceps 70 are arranged in the end portion of the shaft 60 in the positive direction of the Z-axis. The forceps 70 are configured to be opened and closed by a driving force transmitted by the power transmission portion 51. The configuration for the opening/closing action of the forceps 70 is not limited to a particular configuration.

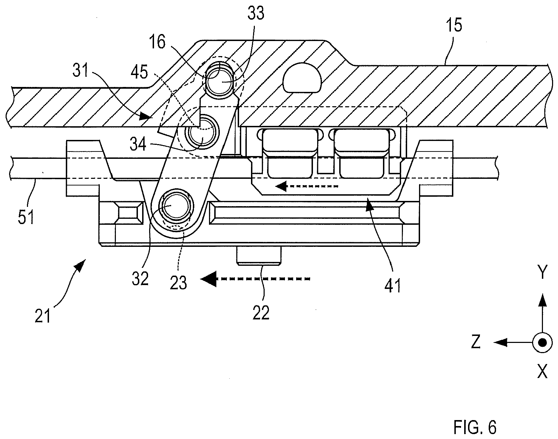

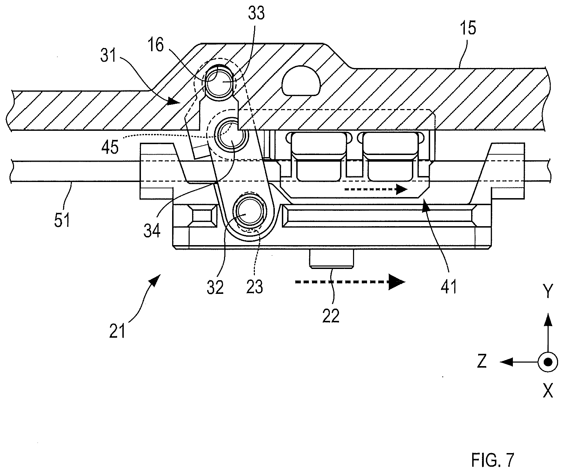

Next, a description will be given of an operation of the surgical tool 1 comprising the above-described configuration with reference to FIG. 6 and FIG. 7. First, the operation when the driven portion 21 is moved in the positive direction of the Z-axis will be described with reference to FIG. 6, and then the operation when the driven portion 21 is moved in the negative direction of the Z-axis will be described with reference to FIG. 7.

As shown in FIG. 6, when the driven portion 21 is linearly moved in the positive direction of the Z-axis by a driving force transmitted from the surgical robot, the motion of the driven portion 21 is transmitted to the conversion portion 31. Specifically, the conversion portion 31 is rotated about the support shaft 33 such that its end adjacent to the driven portion 21 moves in the positive direction of the Z-axis. An amount of movement of the driven shaft 32 of the conversion portion 31 to move in the positive direction of the Y-axis caused by the rotation is absorbed by the driven side opening 23 of the driven portion 21.

When the conversion portion 31 is rotated, the motion of the conversion portion 31 is transmitted to the connection portion 41 and the power transmission portion 51. Specifically, the rotation of the conversion portion 31 is converted into movement of the connection portion 41 and the power transmission portion 51 in the positive direction of the Z-axis through the connection shaft 34 of the conversion portion 31 and the connection hole 46 of the connection portion 41 and transmitted.

At this time, the amount of movement of the connection portion 41 and the power transmission portion 51 in the positive direction of the Z-axis is reduced to a value obtained by multiplying the amount of movement of the driven portion 21 in the positive direction of Z-axis by D11 and dividing the resulting value by D 11+D 12. Moreover, the magnitude of the driving force acting on the connection portion 41 and the power transmission portion 51 in the positive direction of the Z-axis is increased to a value obtained by multiplying the magnitude of the driving force acting on the driven portion 21 in the positive direction of Z-axis by D11+D12 and dividing the resulting value by D11.

As shown in FIG. 7, when the driven portion 21 is linearly moved in the negative direction of the Z-axis by a driving force transmitted from the surgical robot, the conversion portion 31 is rotated about the support shaft 33 such that its end adjacent to the driven portion 21 moves in the negative direction of the Z-axis. The rotation of the conversion portion 31 is converted into movement of the connection portion 41 and the power transmission portion 51 in the negative direction of the Z-axis through the connection shaft 34 of the conversion portion 31 and the connection hole 46 of the connection portion 41 and transmitted.

The amount of movement of the connection portion 41 and the power transmission portion 51 in the negative direction of the Z-axis and the magnitude the driving force acting in the negative direction at this time change in the same manner as in the case shown in FIG. 6; accordingly the detailed description of such changes will not be repeated.

According to the surgical tool 1 configured as described above, the provision of the conversion portion 31 enables the amount of movement of the driven portion 21 in the linear motion direction to be converted and transmitted to the power transmission portion 51. For example, it is possible to set the ratio of conversion by the conversion portion 31 depending on the size of, for example, the forceps 70. Specifically, the conversion ratio is set such that the amount of movement to be transmitted from the driven portion 21 to the power transmission portion 51 is reduced based on the extent of a decrease in size of the forceps 70.

This makes it easier, even when the size of, for example, the forceps 70 is changed, to maintain the relationship between the amount of movement transmitted from the surgical robot to the driven portion 21 and the amount of operation of the forceps 70. In the case where the surgical robot comprises a sensor to detect an external force applied to the forceps 70 and/or a sensor, such as an encoder, to detect a driven amount of the forceps 70, it is possible to inhibit fluctuations in the S/N ratio and fluctuations in the resolution due to a change in size of, for example, the forceps 70. This enables smooth control of the operation of the forceps 70 and inhibits deterioration in accuracy in estimating an external force applied to the forceps 70.

As a result, it is easier to achieve safety and to inhibit complications in robot surgery with the surgical tool 1 of the present embodiment. In addition, this facilitates improvement in QOL (Quality of Life) of patients and reduction of burden on doctors during surgery. Furthermore, this facilitates improvement in the learning curve in robot surgery with the surgical tool 1 of the present embodiment.

The provision of the connection portion 41 makes transmission of a driving force to the power transmission portion 51 easier as compared to a case where a driving force is directly transmitted from the conversion portion 31 to the power transmission portion 51. This also makes the setting easier for converting the amount of movement in the linear motion direction into a specified converted amount.

Forming the conversion portion 31 into the shape described in the present embodiment makes the setting easier for reducing the amount of movement in the linear motion direction at a specified conversion ratio. In other words, it is possible to change the conversion ratio of the amount of movement in the linear motion direction by changing the ratio between the first distance D11, which is from the position at which the conversion portion 31 is rotatably supported with respect to the support portion 15 to the position at which the connection portion 41 is rotatably supported, and the second distance D12, which is from the position at which the conversion portion 31 is rotatably supported with respect to the driven portion 21 to the position at which the connection portion 41 is rotatably supported.

### [Second Embodiment]

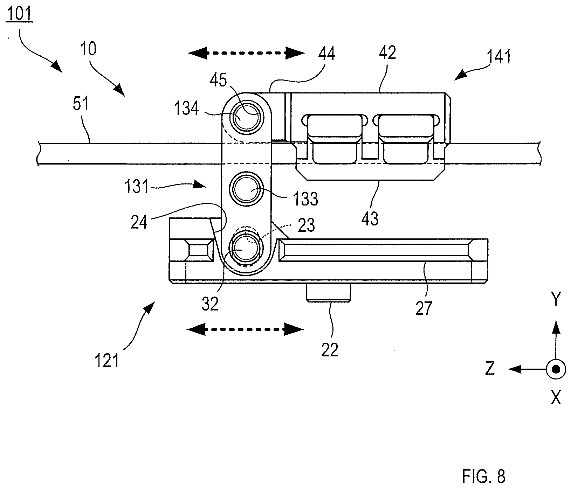

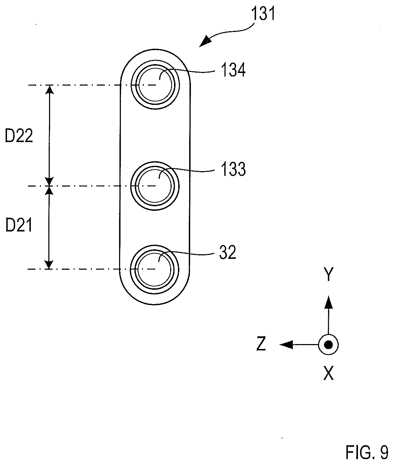

Next, a description will be given of a surgical tool according to a second embodiment of the present disclosure with reference to FIG. 8 and FIG. 9. The basic configuration of the surgical tool according to the present embodiment is the same as in the first embodiment. However, the configuration of the conversion portion and the surrounding thereof is different from the first embodiment. Accordingly, a description will be given with regard to the configuration of the conversion portion and the surrounding thereof with reference to FIG. 8 and FIG. 9, and the description of other configurations will not be repeated in the present embodiment.

As shown in FIG. 8, the body 10 of a surgical tool 101 according to the present embodiment comprises a driven portion 121, a conversion portion 131, a connection portion 141, and the power transmission portion 51. In the present embodiment, the connection portion 141 is arranged at the end of the conversion portion 131 in the positive direction of Y-axis, which is different from the first embodiment.

The driven portion 121 comprises the protrusion 22, the driven side openings 23, and the slide guides 27. The driven portion 121 also comprises the cutout portions 24. In the present embodiment, the driven portion 121 does not comprise the guide portions 25. However, the configuration of the driven portion 121 is not limited to the configuration without the guide portions 25, and the driven portion 121 may comprise the guide portions 25.

The conversion portion 131 forms a linkage mechanism or a lever mechanism that transmits a driving force transmitted to the driven portion 121 to the connection portion 141. The linkage mechanism or the lever mechanism of the present embodiment comprising the conversion portion 131 reduces or increases the amount of movement of the driven portion 121 along the linear motion direction, which is along the Z-axis direction, and transmits the reduced or increased amount of movement to the connection portion 141. The linkage mechanism or the lever mechanism also increases or reduces the magnitude of a driving force in the driven portion 121 and transmits the driving force with the increased or reduced magnitude to the connection portion 141.

The conversion portion 131 is an elongated member extending at least along the Y-axis direction. In the end portion of the conversion portion 131 on the negative direction side of the Y-axis, that is in the end portion adjacent to the driven portion 121, the columnar driven shaft 32 is arranged to extend along the X-axis direction. In the end portion of the conversion portion 131 on the positive direction side of the Y-axis, the columnar connection shaft 34 is arranged to extend along the X-axis direction.

Between the driven shaft 32 and the connection shaft 34 of the conversion portion 131, a columnar support shaft 133 supporting the conversion portion 131 is arranged to extend along the X-axis direction. The support shaft 133 is rotatably held with respect to the support portion 15 of the body 10.

As shown in FIG. 9, in the conversion portion 131, the distance from the center of the driven shaft 32 to the center of the support shaft 133 is a first distance D21. Moreover, in the conversion portion 131, the distance from the center of the support shaft 133 to the center of the connection shaft 134 is a second distance D22.

As shown in FIG. 8, the connection portion 141 transmits a driving force transmitted from the conversion portion 131 to the power transmission portion 51. In comparison with the connection portion 41 in the first embodiment, the connection portion 141 is connected to the connection shaft 34 of the conversion portion 131 such that the connection shaft 34 is rotatable. Since this is the only difference, a detailed description of identical configurations will be omitted.

Next, a description will be given of an operation of the surgical tool 101 comprising the above-described configuration with reference to FIG. 8.

When the driven portion 121 is linearly moved in the positive direction of the Z-axis by a driving force transmitted from the surgical robot, the motion of the driven portion 121 is transmitted to the conversion portion 131. Specifically, the conversion portion 131 is rotated about the support shaft 133 such that its end adjacent to the driven portion 121 moves in the positive direction of the Z-axis.

When the conversion portion 131 is rotated, the motion of the conversion portion 131 is transmitted to the connection portion 141 and the power transmission portion 51. Specifically, the rotation of the conversion portion 131 is converted into movement of the connection portion 141 and the power transmission portion 51 in the negative direction of the Z-axis and transmitted.

At this time, the absolute value of the amount of movement of the connection portion 141 and the power transmission portion 51 in the negative direction of the Z-axis is a value obtained by multiplying the absolute value of the amount of movement of the driven portion 121 in the positive direction of the Z-axis by D22 and dividing the resulting value by D21. Moreover, the magnitude of the driving force acting on the connection portion 41 and the power transmission portion 51 in the positive direction of the Z-axis is a value obtained by multiplying the magnitude of the driving force acting on the driven portion 21 in the positive direction of the Z-axis by D21 and dividing the resulting value by D22.

At this time, if the first distance D21 is greater than the second distance D22, the absolute value of the amount of movement of the connection portion 141 and the power transmission portion 51 in the negative direction of the Z-axis becomes smaller than the absolute value of the amount of movement of the driven portion 121 in the positive direction of the Z-axis. The magnitude of the driving force acting on the connection portion 41 and the power transmission portion 51 in the positive direction of the Z-axis becomes larger than the magnitude of the driving force acting on the driven portion 21 in the positive direction of the Z-axis.

If the first distance D21 is smaller than the second distance D22, the absolute value of the amount of movement of the connection portion 141 and the power transmission portion 51 in the negative direction of the Z-axis becomes larger than the absolute value of the amount of movement of the driven portion 121 in the positive direction of the Z-axis. The magnitude of the driving force acting on the connection portion 41 and the power transmission portion 51 in the positive direction of the Z-axis becomes smaller than the magnitude of the driving force acting on the driven portion 21 in the positive direction of the Z-axis.

The movement of the conversion portion 131, the connection portion 141, and the power transmission portion 51 when the driven portion 121 is linearly moved in the negative direction of the Z-axis by a driving force transmitted from the surgical robot is in a direction opposite to the direction of the movement described above; accordingly a detailed description of such movement will be omitted. Moreover, the amount of movement of the connection portion 141 and the power transmission portion 51 along the Z-axis direction and the magnitude the driving force change in the same manner; accordingly the detailed description of such changes will not be repeated.

According to the configuration described above, forming the conversion portion 131 into the shape as in the present embodiment makes the setting easier for reducing the amount of movement in the linear motion direction or for increasing the amount of movement at a specified conversion ratio. In other words, it is possible to change the conversion ratio of the amount of movement in the linear motion direction by changing the ratio between the first distance D21, which is from the position at which the conversion portion 131 is rotatably supported with respect to the driven portion 121 to the position at which the conversion portion 131 is rotatably supported with respect to the support portion 15, and the second distance D22, which is from the position at which the conversion portion 131 is rotatably supported with respect to the connection portion 141 to the position at which the conversion portion 131 is rotatably supported with respect to the support portion 15.

Setting the first distance D21 greater than the second distance D22 in the conversion portion 131 enables the amount of movement of the driven portion 121 in the linear motion direction to be converted into a smaller amount and transmitted to the power transmission portion 51. Moreover, the magnitude of a driving force is converted into a larger magnitude to be transmitted to the power transmission portion 51.

Setting the first distance D21 smaller than the second distance D22 in the conversion portion 131 enables the amount of movement of the driven portion 121 in the linear motion direction to be converted into a larger amount and transmitted to the power transmission portion 51. Moreover, the magnitude of a driving force is converted into a smaller magnitude to be transmitted to the power transmission portion 51.

It is to be noted that the technical scope of the present disclosure is defined by the appended claim and should not be limited to the above-described embodiments, and various modifications thereto may be made without departing from the intent of the present disclosure. For example, in the embodiments described above, the end effector arranged at the leading end of the shaft 60 is the pair of forceps 70. The end effector, however, is not limited to the forceps 70 and may be other instruments used for, for example, endoscope surgery.

In addition, specific shapes of the conversion portions 31 and 131 are not limited to those described in the above-described embodiments. Any shapes that would achieve the same effect may be used, and thus the shapes are not limited to particular shapes.

## Claims

1. A surgical tool for medical robots (1) comprising:
a driven portion (21) configured to move upon receipt of an external driving force for moving in a linear motion direction;
a power transmission portion (51) configured to transmit the driving force for moving in the linear motion direction to a treatment portion (70) configured to perform a medical treatment;
a conversion portion (31) configured to convert an amount of movement of the driven portion (21) in the linear motion direction to transmit the amount of movement converted to the power transmission portion (51);
a body (10) storing therein the driven portion (21) and the conversion portion (31), and supporting the treatment portion (70); and
a connection portion (41) arranged between the power transmission portion (51) and the conversion portion (31), and configured to transmit the driving force transmitted from the conversion portion (31) to the power transmission portion (51),
the conversion portion (31) being formed in an elongated shape comprising a first end and a second end, the first end being rotatably supported with respect to the driven portion (21) via a driven side opening (23) that is provided in the driven portion (21) and has an oval shape with its long axis extending in a facing direction, and the second end being rotatably supported with respect to a support portion (15) supporting the conversion portion (31) via a support groove (16) that is open in the facing direction of the support portion (15), the facing direction being a direction in which the driven portion (21) and the support portion (15) face each other, and
the connection portion (41) being rotatably supported between the first end and the second end of the conversion portion (31).

## Patentansprüche

1. Chirurgisches Werkzeug für Medizinroboter (1), enthaltend:
einen angetriebenen Abschnitt bzw. Abtriebsabschnitt (21), der konfiguriert ist, sich bei Empfang einer externen Antriebskraft zum Bewegen in einer linearen Bewegungsrichtung zu bewegen;
einen Kraftübertragungsabschnitt (51), der konfiguriert ist, die Antriebskraft zum Bewegen in der linearen Bewegungsrichtung auf einen Behandlungsabschnitt (70) zu übertragen, der konfiguriert ist, eine medizinische Behandlung durchzuführen;
einen Umwandlungsabschnitt (31), der konfiguriert ist, einen Bewegungsbetrag des angetriebenen Abschnitts (21) in die lineare Bewegungsrichtung umzuwandeln, um den umgewandelten Bewegungsbetrag zu dem Kraftübertragungsabschnitt (51) zu übertragen;
einen Körper (10), der den angetriebenen Abschnitt (21) und den Umwandlungsabschnitt (31) darin unterbringt und den Behandlungsabschnitt (70) stützt; und
einen Verbindungsabschnitt (41), der zwischen dem Kraftübertragungsabschnitt (51) und dem Umwandlungsabschnitt (31) angeordnet ist und der konfiguriert ist, die von dem Umwandlungsabschnitt (31) auf den Kraftübertragungsabschnitt (51) übertragene Antriebskraft zu übertragen,
wobei der Umwandlungsabschnitt (31) in einer langgestreckten Form ausgebildet ist, die ein erstes Ende und ein zweites Ende enthält, wobei das erste Ende in Bezug auf den angetriebenen Abschnitt (21) über eine Abtriebsseiten-Öffnung (23) drehbar gestützt ist, die in dem angetriebenen Abschnitt (21) bereitgestellt ist und eine ovale Form aufweist, deren Längsachse sich in einer Blickrichtung erstreckt, und wobei das zweite Ende in Bezug auf einen Stützabschnitt (15) drehbar gestützt ist, der den Umwandlungsabschnitt (31) über eine Stütznut (16) stützt, die in der Blickrichtung des Stützabschnitts (15) offen ist, wobei die Blickrichtung eine Richtung ist, in der der angetriebene Abschnitt (21) und der Stützabschnitt (15) einander gegenüberliegend angeordnet sind, und
wobei der Verbindungsabschnitt (41) zwischen dem ersten Ende und dem zweiten Ende des Umwandlungsabschnitts (31) drehbar gestützt ist.

## Revendications

1. Un outil chirurgical pour robots médicaux (1) comprenant :
une partie entraînée (21) configurée pour se déplacer à la réception d'une force motrice externe pour se déplacer dans une direction de mouvement linéaire ;
une partie de transmission de puissance (51) configurée pour transmettre la force motrice pour un déplacement dans la direction du mouvement linéaire à une partie de traitement (70) configurée pour effectuer un traitement médical ;
une partie de conversion (31) configurée pour convertir une quantité de mouvement de la partie entraînée (21) dans la direction du mouvement linéaire afin de transmettre la quantité de mouvement convertie à la partie de transmission de puissance (51) ;
un corps (10) qui y loge la partie entraînée (21) et la partie de conversion (31) et qui supporte la partie de traitement (70) ;
une partie de connexion (41) disposée entre la partie de transmission de puissance (51) et la partie de conversion (31), et configurée pour transmettre la force motrice transmise de la partie de conversion (31) à la partie de transmission de puissance (51),
la partie de conversion (31) étant formée d'une forme allongée comprenant une première extrémité et une deuxième extrémité, la première extrémité étant soutenue par rotation par rapport à la partie entraînée (21) par une ouverture de côté entraîné (23) qui est prévue dans la partie entraînée (21) et qui a une forme ovale avec son grand axe s'étendant dans une direction de face, et la deuxième extrémité étant supportée par rotation par rapport à une partie de support (15) supportant la partie de conversion (31) par une rainure de support (16) qui est ouverte dans la direction de face de la partie de support (15), la direction de face étant une direction dans laquelle la partie entraînée (21) et la partie de support (15) se font face, et
la partie de connexion (41) étant supportée de manière rotative entre la première et la deuxième extrémité de la partie de conversion (31).
